# FASCICULE DE BREVET EUROPEEN

(11) **EP 1 483 369 B1**
(45) Date de publication et mention de la délivrance du brevet: **10.09.2008**
(21) Numéro de dépôt: 03725273.1
(22) Date de dépôt: 07.03.2003
(51) Int. Cl.: C12N 5/06, C12N 5/10

(54) **LIGNÉES DE CELLULES AVIAIRES UTILES POUR LA PRODUCTION DE SUBSTANCES D'INTÉRÊT**
VÖGELZELL-LINIEN UND DEREN VERWENDUNG ZUR HERSTELLUNG INTERESSANTER BIOLOGISCHER SUBSTANZEN
AVIAN CELL LINES FOR THE PRODUCTION OF USEFUL SUBSTANCES

(30) Priorité: 08.03.2002 FR 0202945
(43) Date de publication de la demande: 08.12.2004
(73) Titulaire: Vivalis, 49450 Roussay (FR)
(72) Inventeur: PAIN, Bertrand, F-69003 Lyon (FR); GUEHENNEUX, Fabienne, 44700 Orvault (FR)
(74) Mandataire: Warcoin, Jacques
(86) Numéro de dépôt international: PCT/FR2003/000735
(87) Numéro de publication internationale: WO 2003/076601

(56) Documents cités:
- WO-A-96/12793
- WO-A-99/06533
- PAIN B ET AL: "LONG-TERM IN VITRO CULTURE AND CHARACTERIZATION OF AVIAN EMBRYONIC STEM CELLS WITH MULTIPLE MORPHOGENETIC POTENTIALITIES" DEVELOPMENT, COMPANY OF BIOLOGISTS, CAMBRIDGE,, GB, vol. 122, 1996, pages 2339-2348, XP002164792 ISSN: 0950-1991 cité dans la demande
- KARAGENC L ET AL: "Soluble factors and the emergence of chick primordial germ cells in vitro." POULTRY SCIENCE, vol. 79, no. 1, janvier 2000 (2000-01), pages 80-85, XP008010610 ISSN: 0032-5791
- BERGER C N ET AL: "Self renewal of embryonic stem cells in the absence of feeder cells and exogenous leukaemia inhibitory factor." GROWTH FACTORS (CHUR, SWITZERLAND) SWITZERLAND 1997, vol. 14, no. 2-3, 1997, pages 145-159, XP008010580 ISSN: 0897-7194
- PAIN B ET AL: "Chicken embryonic stem cells and transgenic strategies." CELLS TISSUES ORGANS, vol. 165, no. 3-4, 1999, pages 212-219, XP000882175 ISSN: 1422-6405 cité dans la demande

## Description

La présente invention se rapporte à un procédé d'obtention de lignées cellulaires aviaires, notamment des cellules souches aviaires, comprenant un sevrage progressif des facteurs de croissance, du sérum ou du tapis nourricier. Ces lignées établies spontanément sont des cellules adhérentes ou non-adhérentes capables de proliférer indéfiniment dans un milieu de culture de base. L'invention porte aussi sur les cellules dérivant de telles lignées qui sont particulièrement utiles pour la production de vaccins et de substances d'intérêt.

Les cellules souches sont des cellules identifiées par leur mise en culture *in vitro* à partir d'un embryon, d'une partie d'un embryon ou même d'un tissu adulte. Par cellule souche, on entend toute cellule pluripotente d'origine embryonnaire ou adulte qui présente une capacité d'autorenouvellement et capable de donner des cellules différenciées spécialisées. En d'autres termes, toute cellule non cancéreuse capable de se diviser indéfiniment en culture et de donner une cellule fille présentant la même capacité de prolifération et de différenciation que la cellule mère dont elle est issue. Ces cellules isolées présentent des caractéristiques morphologiques et immuno-cytochimiques particulières. On peut également distinguer la notion de :
- Cellules souches embryonnaires (cellules CES), cellules souches qui ont la particularité d'être obtenues à partir de la mise en culture de parties ou de l'ensemble d'un embryon très précoce (stade blastula). Ces cellules CES présentent *in vitro* toutes les caractéristiques d'une cellule souche, et *in vivo* la capacité unique de contribuer à la morphogenèse d'un embryon et de participer à la colonisation germinale quand elles sont réimplantées de quelque manière que cela soit dans un embryon receveur.
- Cellules souches somatiques (CSS), cellules qui ont toutes les caractéristiques des cellules souches lorsqu'elles sont cultivées *in vitro*, mais qui contrairement aux cellules CES n'ont pas la potentialité de coloniser *in vivo* les gonades après injection dans un embryon. Elles contribuent uniquement à la morphogénèse des tissus somatiques dans l'embryon.

A l'inverse des cellules primaires déjà différenciées, les cellules souches ne présentent pas un état de différenciation morphologique caractéristique facilement identifiable (fibroblaste, adipocyte, macrophage, etc...), mais sont plutôt caractérisées par un état de prolifération et de non différenciation. Cet état se traduit par différents comportements comme une prolifération rapide *in vitro*, une morphologie caractéristique, la présence de différents marqueurs, des besoins en facteurs de croissance variables et une aptitude à répondre à des stimuli d'induction de différenciation particuliers. Elles ne sont pas sensibles à la sénescence réplicative, période critique pour un grand nombre de cellules primaires différenciées, dont les fibroblastes par exemple.

Pour le maintien *in vitro* des cellules souches aviaires pendant des périodes de temps importantes, il faut respecter des conditions de culture et d'entretiens spécifiques telles que décrites dans Pain et al., 1996; US 6,114,168 et EP 787 180.

Ces documents brevets, dont le contenu est équivalent au contenu de la demande internationale de brevet publiée sous le numéro WO 96/12793, décrivent en particulier un milieu de culture de cellules embryonnaires totipotentes aviaires comprenant des facteurs de croissance et des cytokines, ce milieu étant substantiellement dépourvu d'acide rétinoïque actif.

On peut également citer la demande internationale de brevet publiée sous le numéro WO 99/06534 qui décrit un milieu de culture pour la production de lignées cellulaires germinales embryonnaires aviaires, ce milieu comprenant notamment comme facteurs de croissance du LIF (facteur inhibant la leucémie), bFGF (facteur de croissance de fibroblaste de base), IGF (facteur de croissance apparente à l'insuline) et du SCF (facteur de cellule souche).

On peut aussi citer la demande internationale de brevet publiée sous le numéro WO 00/47717 qui décrit en particulier un procédé d'établissement de lignées cellulaires embryonnaires germinales aviaires à partir de cellules germinales primordiales cultivées dans un milieu comprenant notamment des facteurs de croissance comme le SCF, le bFGF, l'IGF-1 ou encore 1'IL-11 (Interleukine 11), et un facteur inhibant la différentiation comme le LIF.

La mise en culture *in vitro* d'une cellule primaire dans des conditions satisfaisantes de milieu et de facteurs de croissance lui permet de proliférer seulement pendant un certain nombre de passage. Cette cellule peut être obtenue directement d'un tissu dissocié ou d'une partie de ce tissu. Ce nombre de passage est néanmoins très dépendant de l'espèce animale considérée, de l'origine du tissu, de l'âge de l'organisme donneur, etc.... Dans la plupart des cas, la prolifération cellulaire observée *in vitro* ralentit progressivement puis les cellules cessent de proliférer.

Cet arrêt correspond souvent à une sénescence réplicative, connue sous le terme de limite de Hayflick. Cet arrêt serait le résultat de l'action d'une véritable horloge moléculaire dont un des acteurs clefs serait la longueur des télomères. Les télomères sont des séquences répétées situées à l'extrémité des chromosomes. Le raccourcissement de ces structures nucléotidiques répétitives est la conséquence de la réplication du DNA sur un mode semi conservatif. En l'absence de l'enzyme télomérase, en charge d'ajouter les séquences répétées à l'extrémité des chromosomes, un point de non-retour est atteint quant à la taille des télomères, point au-delà duquel est déclenché un mécanisme moléculaire encore inconnu d'activation de gènes impliqués dans le contrôle du cycle cellulaire. Les cellules seraient alors bloquées en phase G1 dans leurs divisions et cesseraient de proliférer. De nombreux acteurs semblent intervenir dans ce contrôle négatif du cycle cellulaire comme les différentes cyclines, les kinases spécifiques, les protéines RB, P53, les facteurs de transcription spécifiques comme E2F et bien d'autres (mdm2, BTG, p21,...). A l'inverse, l'enzyme télomérase peut donc être vue comme un acteur central de l'immortalité cellulaire, car elle maintient la longueur des télomères et donc rend insensible la cellule à cette perte engendrée par les divisions successives.

Dans un organisme en développement et au cours de la vie de cet organisme, seuls quelques types cellulaires, dont certains lymphocytes présentent une expression permanente de la télomérase. Cette activité semble également être une des caractéristiques des cellules souches, à la fois au niveau somatique (CSS) et au niveau germinal. Cette propriété d'expression, de maintien d'expression ou de 'réveil' d'expression de l'activité télomérase est également souvent associée au caractère immortel d'une cellule maintenue *in vitro*. A ce jour, de nombreuses cellules cancéreuses sont également détectées positives pour l'activité télomérase. Cette activité serait en partie responsable de la capacité de prolifération incontrôlée des cellules tumorales *in vivo*.

Cette activité télomérase est, dans tous les cas, un excellent marqueur du caractère cellule souche ainsi que du lignage germinal et de la capacité d'une cellule à devenir immortelle. Deux critères sont donc utilisés : l'activité de la télomérase et la taille des télomères.

Dans une perspective d'établissement et de façon très schématique, d'après la littérature et les résultats déjà disponibles dans de nombreux laboratoires, l'établissement de lignées cellulaires peut être réalisé selon deux voies : un établissement spontané résultant de dommages génétiques intrinsèques non induits ou un établissement provoqué, induit par l'utilisation de virus, rétrovirus ou par d'autres moyens tels que des agents chimiques, des irradiations, des U.V (Ultra-Violet).... Au niveau des mammifères, par exemple, l'établissement des cellules de rongeurs (souris, rat, ...) est reconnu comme assez facile de façon spontanée, par contre, la situation est toute autre pour les cellules humaines, quelque soit leur origine tissulaire (Smith and Pereira-Smith, 1996).

Ainsi, lorsque l'on souhaite dériver des lignées cellulaires des cellules souches aviaires mentionnées ci-dessus en vue de produire en masse des substances d'intérêt *in vitro,* le problème est de pouvoir maintenir des cellules en culture dans un milieu économique tout en évitant les écueils tels que la différenciation et la sénescence cellulaire.

Au niveau aviaire, il est en général admis que l'établissement de cellules primaires est un événement rare, voire quasi inexistant. La seule exception notable publiée semble être la lignée DF-1 qui résulte de l'immortalisation décrite comme spontanée de fibroblastes de poulet (Foster et al., 1991 ; brevet US 5,672,485, N° ATCC CRL 12203). Au niveau de cette lignée, des articles récents mentionnent les premiers éléments de dérégulation observée (Kim et al., 2001 a ; Kim et al., 2001b).

Dans le processus d'immortalisation, une première étape conduit la cellule en prolifération vers la limite de Hayflick, qui selon les types cellulaires se situe entre 10 et 50 passages. Un premier événement mutationnel spontané a alors lieu qui permet à la cellule de franchir ce premier blocage, événement qui affecte souvent les gènes p53, pRb, ... .Les cellules continuent donc de proliférer jusqu'au moment où un deuxième blocage intervient qui est en général levé par de nouvelles mutations dans d'autres gènes et par l'activation de la télomérase, souvent observée.

Au niveau aviaire, il est en général admis que l'établissement de cellules primaires immortelles est un événement quasi inexistant. C'est pourquoi un nombre important de lignées ont été obtenues par la mise en culture de cellules tumorales, souvent directement prélevées à partir d'une biopsie de la tumeur. Cette obtention *in vitro* est en fait le résultat de l'altération *in vivo* de certains gènes, responsables de l'apparition de la tumeur. Un exemple est fourni par la lignée fibroblastique SB-CEV-1, isolée à partir de la mise en culture d'une tumeur viscérale d'un animal. (ATCC N° CRL 10497, brevet US N° 5,846,527). Cette approche est grandement facilitée par l'existence d'un très grand nombre de virus et rétrovirus aviaires, identifiés, isolés et souvent caractérisés au niveau moléculaire. Souvent oncogéniques par leur action directe ou indirecte (activation d'un gène endogène transformant lors de leur intégration, expression de la (ou les) protéine oncogénique endogène au virus), ces virus provoquent des tumeurs, des lésions, ainsi l'obtention de lignées cellulaires dans différents lignages différenciés est possible à partir de la mise en culture des organes infectés des animaux. L'utilisation *in vitro* de ces virus et rétrovirus, isolés *in vivo*, a été développée de façon complémentaire. On peut ainsi citer de façon non exhaustive :
- les lignées lymphoblastoïdes DT40 et DT95, obtenues en présence du virus de la leucose aviaire (ALV) et dans lesquelles le locus myc est activé (Baba et al., 1985, ATCC, N°CRL 2111, CRL N° 2112) ,
- la lignée lymphoblastoïde de dinde MDTC-RP19, établie avec le virus de la maladie de Marek (US N° 4,388,298, ATCC N° 8135),
- la lignée lymphoblastoïde ConA-C1, établie avec le virus REV (Reticuloendothelial virus, ATCC N° 12135, US N° 5,691,200)
- la lignée myélomonocytaire BM2 établie avec le virus MH2 (Liu et al., 1977, brevet US N°5,388,680),
- et toute une série de lignées hématopoiétiques obtenues avec différents virus,
   ○ la lignée érythroblastique HD4 (6C2), obtenue avec le virus AEV
   ○ la lignée monocytaire HD11 (Beug et al., 1979), obtenue avec le virus MC29
   ○ la lignée granulocytaire HD13 (Golay et al., 1988), obtenue avec le virus E26
   ○ la lignée hématopoiétique mixte HD57 (Metz and Graf, 1991) également obtenue avec le virus E26

Cependant, le problème dans ces approches de transformation est l'obtention de cellules qui produisent des virus et portent le génome activé des virus et rétrovirus utilisés. Ces activations et cette présence virale sont des freins à leur utilisation industrielle comme support de réplication pour des virus d'intérêt ou pour la production de protéines spécifiques dans des conditions optimales de sécurité.

Une autre approche de sur-expression d'oncogènes, de gènes immortalisants (gène E1A de l'adénovirus, 'large T' du polyome SV40, etc...) ou de fragments de gènes a également permis d'obtenir des lignées à partir de cellules primaires déjà différenciées. Ces éléments peuvent être introduits dans les cellules par simple transfection de vecteur permettant l'expression de la partie immortalisante, mais également introduits via les virus ou rétrovirus modifiés génétiquement pour exprimer ces éléments immortalisants. L'origine des immortalisants peut être aviaire ou non, virale ou non. Le tropisme pour les cellules aviaires pouvant être d'ailleurs lié au virus originel ou également modifié. A titre d'exemple, la lignée TDF-2A de fibroblastes de canard est ainsi obtenue par introduction d'un premier gène immortalisant puis d'un gène anti-apoptotique (Guilhot et al., 1993, brevet US 6,255,108). D'autres méthodes ont été développées, comme la sur-expression de p53 (Foster et al., brevet US N° 5,830,723).

En outre, l'action de carcinogènes chimiques directement sur l'animal selon différents modes d'administration a permis entre autres l'obtention
- des lignées QT6 et QT35, de fibroblastes de caille (Moscovici et al., 1977, ATCC N° CRL 1708),
- de la lignée d'hépatocyte de poulet LMH (Kawaguchi et al., 1989, ATTC N° CRL 2117),
- de la lignée CHCC-OU2 de fibroblastes de poulet (ATCC, N° CRL 12302, Brevet US N° 5,989,805).

Par événement d'immortalisation, on entend différentes actions comme :
- l'action de stress oxydatifs, thermiques, chimiques capables d'induire des modifications de la physiologie des cellules et/ou des mutations,
- l'action des produits de gènes spécifiques dans la physiologie des cellules comme certains gènes immortalisants (oncogènes, proto-oncogènes, gènes du cycle cellulaire, gènes anti-apoptiques, etc...),
- la destruction ciblée par recombinaison ou inactivation fonctionnelle d'anti-oncogènes, de gènes apoptiques, de gènes suppresseurs de tumeurs, de gènes anti-prolifératifs menant à une dérégulation fonctionnelle du cycle cellulaire ou de la physiologie de la cellule,
- le contrôle de gènes de prolifération par leur blocage fonctionnel, etc...,
- l'action de rayonnements (UV, gamma, X, etc...),
- l'action de mutagènes chimiques (substances endommageant l'ADN, substances analogues de facteurs de croissance, etc...),
- l'action conjuguée de ces différentes actions prises séparément.

Dans le cadre de l'invention, on a trouvé que le sevrage des facteurs de croissance, du sérum et/ou du tapis nourricier conduit à l'isolement de populations de cellules souches pouvant croître indéfiniment dans des milieux de culture de base.

En outre, hormis les cellules souches hématopoiétiques qui sont pour la plupart des cellules non adhérentes, la majorité des cellules, obtenues selon les techniques de l'art évoquées ci-dessus (fibroblastes, hépatocytes, etc...), présentent un phénotype adhérent. Or, l'utilisation industrielle des cellules, comme support de réplication virale, privilégie les cellules non adhérentes. Ce phénotype est intéressant tant pour des raisons de facilité de manipulation qui évite l'usage d'enzyme protéolytique de dissociation que pour les densités importantes atteintes par les cellules non-adhérentes cultivées *in vitro*.

La présente invention décrit l'obtention de lignées qui peuvent devenir non-adhérentes spontanément ou pour lesquelles la non adhérence a été obtenue par un sevrage du tapis nourricier. Du fait de leur croissance en suspension, ces lignées sont parfaitement adaptées à une utilisation industrielle pour la production de substances d'intérêt dans des bioréacteurs.

En plus de leurs propriétés à croître sur milieu de culture de base, on a découvert que ces lignées cellulaires permettent la réplication de certains virus à des rendements équivalents voire supérieurs aux rendements obtenus avec les procédés en cours ; ce qui rend ces cellules particulièrement utiles pour la production en masse de vaccins.

### Description

Ainsi, dans un premier aspect, la présente invention se rapporte à un procédé d'obtention de lignées cellulaires aviaires, **caractérisé en ce qu**'il comprend les étapes suivantes :
a) mise en culture de cellules souches embryonnaires aviaires dans un milieu comportant:
   - au moins les facteurs de croissance SCF, IGF-1 et bFGF, et au moins une cytokine choisie parmi le LIF, IL-11, IL-6, IL-6R, CNTF, oncostatine et cardiotrophine; - un tapis inactivé de cellules STO, et
   - du serum de veau foetal à une concentration de 12 à 8%,
b) Après une vingtaine de passages le milieu de culture est modifié par sevrage progressif desdits facteurs de croissance et de ladite cytokine, ou du tapis nourricier, ou par diminution de la concentration en sérum de veau foetal jusqu'à arriver à des faibles proportions de 2%,
c) établissement de lignées cellulaires adhérentes ou non-adhérentes capables de proliférer dans un milieu de base en l'absence de facteurs de croissance exogènes et de cytokine, de sérum et/ou du tapis nourricier inactivé, l'établissement de lignées cellulaires non adhérentes étant obtenus par ensemencement par 1x10⁶ cellules/ml au moins en boite bactériologique suivi de plusieurs passages en dilution simple.

Dans le cadre de l'invention, on entend par « établissement d'une lignée », le maintien de cellules en culture *in vitro* sur une période considérable de temps. Avantageusement, les cellules issues des lignées obtenues à l'étape c) sont capables de proliférer pendant au moins 50 jours, 100 jours, 150 jours, 300 jours ou de préférence au moins 600 jours. Les 600 jours ne constituent pas une limite temporelle car les lignées cellulaires obtenues sont toujours vivantes après des périodes de temps bien plus longues. Dès lors, ces lignées sont considérées comme pouvant croître indéfiniment en milieu de culture de base dépourvu de facteurs de croissance exogènes, de sérum et/ou du tapis nourricier inactivé. Par « lignée », on entend toute population de cellules capables de proliférer indéfiniment en culture *in vitro* en gardant peu ou prou les mêmes caractéristiques morphologiques et phénotypiques.

Les cellules issues des lignées selon l'invention peuvent être des cellules souches aviaires, notamment des cellules embryonnaires souches aviaires, ladite cytokine du milieu de culture à l'étape a) du procédé selon l'invention étant choisie parmi le LIF, l'IL-11, l'IL-6, l'IL-6R, le CNTF, l'onconstatine et la cardiotrophine.

Il est décrit ici que les cellules souches selon l'invention peuvent servir pour dériver des lignées de cellules différenciées. En effet, ces cellules souches présentent la propriété d'être pluripotentes, c'est à dire qu'elles ont les potentialités pour être induites dans de multiples voies de différenciation, caractérisables par différents marqueurs spécifiques.

Ces cellules peuvent également être des cellules précurseurs, qui correspondent aux cellules d'un tissu adulte ou embryonnaire partiellement différenciées par opposition à une cellule souche et qui est capable de se diviser et de donner des cellules plus différenciées. Par « cellule différenciée », on entend toute cellule d'un tissu adulte ou embryonnaire spécialisée, présentant des marqueurs spécifiques ou remplissant des fonctions physiologiques spécifiques. Il est possible, dans un aspect particulier de l'invention, notamment pour des isolats particuliers ou des clones issus d'un isolat particulier obtenu au cours de l'établissement, que ces cellules souches puissent contribuer à la lignée germinale. Dans ce cas, ces cellules souches établies en lignées seraient des cellules souches embryonnaires.

Bien entendu, le procédé mentionné ci-dessus permet d'obtenir des clones cellulaires issus des cellules provenant des lignées établies. Ces clones sont des cellules génétiquement identiques à la cellule de laquelle elles dérivent par division.

Dans un mode de réalisation particulier, l'invention porte sur un procédé tel que défini précédemment, dans lequel les lignées établies sont des cellules souches adhérentes qui prolifèrent en l'absence du tapis nourricier inactivé.

A ce titre, dans le procédé décrit ci-dessus, l'étape b) consiste en un sevrage des composants du milieu (facteurs de croissance et cytokine seulement ou sérum seulement ou facteurs de croissance et cytokine puis sérum ou encore sérum puis facteurs de croissance).

Dans un autre mode de réalisation, l'invention porte sur un procédé tel que défini précédemment dans lequel les lignées établies sont des cellules souches non adhérentes qui prolifèrent en suspension dans un milieu dépourvu de facteurs de croissance exogènes.

A ce titre, dans le procédé décrit ci-dessus, l'étape b) consiste en un sevrage progressif ou total du tapis nourricier puis éventuellement à un sevrage des autres composants du milieux (facteurs de croissance, cytokine et sérum).

Dans un autre mode de réalisation, l'invention porte sur un procédé tel que défini précédemment dans lequel les lignées établies sont des cellules souches non adhérentes qui prolifèrent en suspension dans un milieu de sérum foetal à des proportions de 2%.

Dans un autre mode de réalisation, l'invention porte sur un procédé tel que défini précédemment dans lequel les lignées établies sont des cellules souches non adhérentes qui prolifèrent en suspension dans un milieu dépourvu de facteurs de croissance exogènes et de cytokine et contenant du sérum foetal à des proportions de 2%.

Dans une autre alternative, l'étape b) consiste en un sevrage progressif ou total des facteurs de croissance, optionnellement suivi par une diminution de la concentration en serum de veau foetal jusqu'à arriver à des proportions de 2%. Dans une autre alternative, l'étape b) consiste en un sevrage progressif des facteurs de croissance et de cytokine et/ou à une diminution de la concentration en sérum jusqu'à arriver à 2%, optionnellement suivi par un sevrage du tapis nourricier.

En outre, les lignées établies, peuvent être des cellules qui prolifèrent dans un milieu appauvri en sérum foetal à des proportions de 2%. Par appauvri en sérum on entend une diminution progressive de la concentration en sérum étalée dans le temps. Cette méthode permet une sélection de clones qui s'adaptent à ces nouvelles conditions de plus en plus drastiques jusqu'à l'obtention de lignées stables qui sont capables de croître en milieu appauvri en sérum ou encore complètement dépourvu de sérum.

Le procédé décrit ci-dessus peut comprendre en outre une étape dans laquelle les cellules obtenues à l'étape c) sont soumises à une sélection dans des milieux de cultures utilisés pour la production à grande échelle de sorte à obtenir des clones adaptés à la production de vaccins destinés à la thérapie humaine ou animale.

Les cellules selon l'invention présentent au moins une des caractéristiques suivantes :
- un nucléole bien visible et un très faible cytoplasme,
- une activité alcaline phosphatase endogène,
- une activité télomérase endogène,
- une réactivité avec des anticorps spécifiques sélectionnés parmi le groupe des anticorps SSEA-1 (TEC01), SSEA-3, et EMA-1.

De préférence, les cellules de l'invention présentent l'ensemble des caractéristiques mentionnées ci-dessus.

Il est decrit ici un procédé d'obtention de lignées aviaires mentionné ci-dessus dans lequel les cellules issues des lignées obtenues à l'étape c) sont modifiées pour permettre une meilleure utilisation *in vitro* comme l'extension de la durée de vie ou des densités de croissance plus importantes ou encore des besoins en nutriment moins importants.

Avantageusement, les cellules issues des lignées établies sont modifiées pour produire une substance d'intérêt, notamment un polypeptide d'intérêt, un anticorps ou un virus atténué. Lesdites cellules peuvent être modifiées avec toute technique à la portée de l'homme du métier, notamment la recombinaison homologue, dirigée et/ou conditionnelle (système Cre-Lox ou FLP-FRT), par transformation par tout vecteur, plasmide, notamment à l'aide de rétrovirus.

Le milieu utilisé à l'étape a) comprend au moins une cytokine sélectionnée parmi les LIF, IL-11, IL-6, IL-6R, CNTF, Oncostatine et cardiotrophine, et les facteurs de croissance SCF, IGF-1 et bFGF.

Le tapis nourricier inactivé utilisé à l'étape a) se compose de fibroblastes de souris établies en lignée, les cellules STO qui peuvent ou non être modifiées ou transfectées par des vecteurs d'expression (Pain et al, 1996). Dans ce procédé, les cellules utilisées à l'étape a) sont des cellules obtenues par la mise en suspension de cellules provenant de disques blastodermiques d'oeufs fécondés dans un milieu de culture comprenant au moins une cytokine, b-FGF, et SCF. Lesdites cellules sont ensemencées sur un tapis de cellules nourricières, incubées, puis prélevées.

L'étape b) consiste en un sevrage progressif de chaque facteur de croissance et de cytokine ajoutés dans le milieu de l'étape a), comprenant un passage dans un milieu neuf dépourvu d'au moins un desdits facteurs ou cytokine et à répéter différents passages successifs jusqu'à ce que le milieu soit dépourvu de l'ensemble desdits facteurs et cytokine. Par sevrage progressif, on entend une suppression facteur par facteur dans le milieu de culture. Alternativement, il est décrit que l'on peut procéder à un sevrage drastique ou total, c'est à dire l'enlèvement de l'ensemble desdits facteurs en une seule fois. Ainsi, le sevrage à l'étape b) peut consister à diminuer la concentration d'un ou plusieurs facteurs progressivement ou à mettre en culture les cellules souches aviaires directement dans un milieu dépourvu d'un ou plusieurs facteurs ou encore dépourvu de la totalité desdits facteurs.

L'étape b) peut également comprendre le sevrage du sérum. A ce titre, le sevrage peut être progressif en diminuant la concentration en sérum lors de chaque passage, par exemple en passant de 10 % à 7,5% puis 3,75% et 2%. Alternativement, il est décrit ici que l'on peut procéder à un sevrage drastique.

L'étape b) peut également comprendre le sevrage du tapis nourricier. Le sevrage du tapis nourricier peut également être progressif en diminuant le nombre de cellules nourricières inactivées lors de chaque passage. Alternativement, il est décrit ici que l'on peut procéder à un sevrage drastique.

Bien entendu, l'ordre des sevrages peut varier. Par exemple, on peut commencer par le sevrage des facteurs de croissance et de cytokine, et poursuivre avec le sevrage du tapis nourricier.

Ainsi, dans un autre aspect, l'invention se rapporte aux lignées cellulaires établies et aux cellules issues desdites lignées obtenues à partir du procédé décrit ci-dessus, lesdites cellules étant capables de proliférer pendant au moins 50 jours, 100 jours, 150 jours, 300 jours, ou de préférence au moins 600 jours dans un milieu dépourvu de facteur de croissance exogène et de cytokine appauvri en sérum jusqu'à arriver à 2% et/ou depourvu de tapis nourricier.

Ces lignées cellulaires et les cellules qui en dérivent sont capables de proliférer pendant au moins 50 jours, 100 jours, 150 jours, 300 jours, ou de préférence au moins 600 jours dans un milieu de base, notamment dans un milieu tel que DMEM, GMEM, HamF12 ou McCoy supplémenté de différents additifs couramment utilisés par l'homme du métier. Parmi les additifs, on peut citer les acides aminés non essentiels, les vitamines et le pyruvate de sodium.

L'invention porte également sur les lignées cellulaires et les cellules issues de telles lignées décrites ci-dessus, **caractérisées en ce qu**'elles sont des cellules souches aviaires, en particulier des cellules souches embryonnaires aviaires.

Ces cellules souches peuvent être adhérentes tout en proliférant en l'absence du tapis nourricier inactivé. Alternativement, ces cellules souches sont non adhérentes et prolifèrent en suspension dans un milieu de base évoqué ci-dessus.

Ces cellules se caractérisent également en ce qu'elles présentent au moins une des caractéristiques suivantes :
- un nucléole bien visible et un très faible cytoplasme,
- une activité alcaline phosphatase endogène,
- une activité télomérase endogène,
- une réactivité avec des anticorps spécifiques sélectionnés parmi le groupe des anticorps SSEA-1 (TEC01), SSEA-3, et EMA-1.

Avantageusement, ces cellules sont modifiées génétiquement de sorte à produire une substance d'intérêt, notamment un polypeptide d'intérêt, un anticorps ou un virus atténué.

Lesdites cellules peuvent par exemple supporter la réplication de virus vivants ou atténués, en particulier les virus sélectionnés dans le groupe des adénovirus, des hepadnavirus, des herpesvirus, des orthomyxovirus, des papovavirus, des paramyxovirus, des picornavirus, des poxvirus, des reovirus et des rétrovirus. De préférence, les virus répliqués sur ces cellules appartiennent à la famille des orthomyxovirus, en particulier le virus de la grippe ou à la famille des paramyxovirus, en particulier les virus de la rougeole, des oreillons et de la rubéole.

Ainsi, l'invention porte sur les lignées cellulaires décrites ci-dessus, les cellules dérivant desdites lignées et également des lignées cellulaires obtenues à partir des cellules qui ont été modifiées génétiquement. De préférence, l'invention vise les lignées cellulaires issues de l'étape c) du procédé décrit plus haut **caractérisées en ce qu**'elles sont des cellules souches aviaires capables de croître indéfiniment en milieu de base dépourvu de facteurs de croissance exogènes, et e cytokine, appauvri en sérum jusqu'à arriver à 2% et/ou de tapis nourricier.

Dans un autre aspect de l'invention, les cellules obtenues en fin d'étape c) peuvent être génétiquement modifiées.

Il est décrit ici également une culture cellulaire comprenant des cellules issues des lignées cellulaires décrites ci-dessus, notamment des cellules souches aviaires ou des cellules souches embryonnaires aviaires et un milieu de base dépourvu de facteurs de croissance exogènes, appauvri en sérum ou dépourvu de sérum et/ou du tapis nourricier inactivé.

Dans un aspect supplémentaire, l'invention concerne l'utilisation des lignées cellulaires et des cellules décrites précédemment pour la production de substances d'intérêt en particulier des protéines d'intérêt thérapeutiques, pour la réplication de virus vivants ou atténués, en particulier les virus pris dans le groupe des adénovirus, des hepadnavirus, des herpesvirus, des orthomyxovirus, des papovavirus, des paramyxovirus, des picornavirus, des poxvirus, des reovirus et des rétrovirus.

De préférence, on utilise les lignées cellulaires et les cellules décrites précédemment pour la production de virus appartenant à la famille des orthomyxovirus, en particulier le virus de la grippe et pour la production de virus appartenant à la famille des paramyxovirus, en particulier les virus de la rougeole, des oreillons et de la rubéole.

Dans ces lignées et cellules, utilisées pour supporter la réplication de virus vivants ou atténués, on peut introduire le ou les composants nécessaires à l'accomplissement du cycle viral complet du virus de sorte à obtenir par exemple la sur-expression du récepteur au virus à la surface de la cellule.

Pour la suite de la description, on se référera aux légendes des figures ci-dessous.

### Légendes

**Figures 1-3** **: Courbes de croissance des lignées cellulaires de l'invention** (avec sevrage du sérum (fig. 2) et avec sevrage du tapis nourricier (fig. 3).
**Figures 4** **: Photo présentant la morphologie caractéristique des cellules souches aviaires**
   N : noyau, n : nucléole et C : cytoplasme
   (isolat S86N 99, grossissement X40, photo réalisée avec un appareil numérique Cyber-shot Sony)
**Figures 5** **: Photo présentant l'activité alcaline phosphatase des lignées de cellules souches aviaires adhérentes ou en suspension**

Après fixation (0.1% formaldéhyde/0.5% glutaraldéhyde, 30 minutes à 4°C) les cellules sont rincées deux fois en PBS 1X et incubées entre 10 à 30 minutes à 37°c dans une solution de NBT/BCIP (Nitro Blue Tetrazolium chloride 0.375mg/ml, 5-Bromo-4-Chloro-3-indolyl Phosphate 0.188 mg/ml, 0.1 M Tris pH 9.5, 0.05 M MgCl², 0.1M Nacl). La réaction est arrêtée par deux lavages PBS 1X et les photos sont réalisées.
**A-** illustre la coloration violette caractéristique de l'activité alcaline phosphatase endogène obtenue pour la lignée adhérente S86N45 p87, lignée cultivée sans feeder ni facteur (grossissement X40, appareil numérique Cyber-shot de Sony).
**B-** illustre la coloration violette caractéristique de l'activité alcaline phosphatase endogène obtenue pour la lignée EB14 maintenue depuis 8 passages en suspension, lignée dérivée des cellules S86N 45, cultivée en suspension sans feeder ni facteur (grossissement X20, appareil numérique Cyber-shot de Sony).

### Exemple 1 : origine variable du matériel vivant utilisé

L'établissement de lignées cellulaires est souvent très lié à la nature génétique du matériel cellulaire. Ainsi, dans l'exemple murin, proportionnellement peu de fonds génétiques sont permissifs à l'obtention de cellules souches embryonnaires ES et implique souvent une notion d'animaux consanguins. Au niveau aviaire, il est difficile d'obtenir des animaux consanguins pour des raisons historiques et d'origine de sélection des souches commerciales ; l'intérêt étant d'éviter justement la consanguinité. Les oeufs sont la source initiale des cellules mises en culture dans cette invention. Les oeufs sont utilisés de préférence non incubés, mais quelques heures d'incubation peuvent être nécessaires pour obtenir les premiers stades de développement de l'embryon. Les cellules obtenues sont issues de différentes souches de poulet. Parmi les souches utilisées, on peut citer la souche S86N, souche commerciale destinée à la production de poulet Label, les CNR, souche destinée à la production de poulet Label, les Marens, souche locale bien caractérisée génétiquement et phénotypiqement, les White Leghorn, souche plus destinée à la production d'oeufs de consommation et souche de référence des laboratoires de recherche, etc.... Dans cette dernière souche, différentes origines ont été testées dont certains oeufs (appelés Valo) issus de la souche White Leghorn de chez Lohmann (Allemagne) considérés comme des oeufs 'SPF' (SPF Specific Pathogen Free) maintenues dans des conditions très particulières de sécurité sanitaire. De nombreux isolats de cellules ont été obtenus à partir de différentes souches, suggérant le caractère général de la méthode.

### Exemple 2 : Obtention et établissement des cellules adhérentes

Les oeufs sont ouverts, le jaune est séparé du blanc lors de l'ouverture. Les embryons sont prélevés sur le jaune soit directement à l'aide d'une pipette Pasteur, soit à l'aide d'un petit filtre de papier absorbant (Papier Whatmann 3M), préalablement découpé sous forme d'un anneau perforé à l'aide d'une perforeuse. Le diamètre de la perforation étant d'environ 5 mm. Ces petits anneaux sont stérilisés à la chaleur sèche pendant environ 30 minutes au four. Ce petit anneau de papier est déposé sur la surface du jaune et centré au niveau de l'embryon qui est ainsi entouré par l'anneau de papier. Ce dernier est alors découpé à l'aide de petits ciseaux et l'ensemble prélevé est placé dans une boite de Petri, remplie de PBS ou d'un liquide physiologique. L'embryon ainsi entraîné par l'anneau est nettoyé de l'excès de jaune dans le milieu et le disque embryonnaire, ainsi débarrassé de l'excès du vitellus, est prélevé avec une pipette Pasteur.

Dans les deux cas, les embryons sont mis dans un tube contenant du milieu physiologique (PBS1X, Tris Glucose, milieu, ...). Les embryons sont alors dissociés mécaniquement et ensemencés sur un 'feeder' dans du milieu de culture défini. Parmi les conditions préférentielles utilisées pour les mises en culture, on privilégie le milieu de culture composé du milieu MacCoy comme milieu de base supplémenté avec du sérum de veau foetal à une concentration initiale de 12 à 8 %, avec des acides aminés non essentiels à 1 %, avec un mélange de vitamines d'origine commerciale à 1 %, avec du pyruvate de sodium à une concentration finale de 1mM, avec du beta-mercaptoéthanol à une concentration finale de 0,2 mM, de la glutamine à une concentration finale de 2,9 mM, avec un mélange initial d'antibiotiques contenant de la gentamycine à une concentration finale de 10 ng/ml, de la pénicilline à une concentration finale de 100 U/ml et de la streptomycine à une concentration finale de 100 µg/ml. Rapidement après les premiers passages des cellules, le mélange d'antibiotiques n'est plus ajouté dans le milieu. Par rapidement, on entend après les 3 à 5 premiers passages en général. Un mélange de nucléosides peut être ajouté également, ce mélange étant constitué comme décrit précédemment (Pain et al., 1996). Parmi les milieux de bases testés dans ces mêmes conditions et qui donnent des résultats similaires, on trouve les milieux Ham F12, Glasgow MEM et le DMEM, ce dernier supplémenté en biotine à une concentration finale de 8 mg/l. A titre de comparaison, la concentration de biotine est de 0,2 mg/l dans le milieu MacCoy, de 0, 0073 mg/l dans le Ham F12 et de 0 dans les milieux DMEM et GMEM commerciaux.

Les facteurs de croissance et les cytokines ajoutés dans le milieu de culture sont de façon privilégiée des facteurs et des cytokines recombinantes dont le SCF de souris à une concentration finale de 1ng/ml, l'IGF-1 à une concentration finale de 1 à 5 ng/ml, le CNTF à une concentration finale de 1 ng/ml, l'IL-6 à une concentration finale de 1 ng/ml, et le récepteur soluble de l'IL-6 à une concentration finale de 0,5 ng/ml à 1 ng/ml. Dans certaines expériences, certains autres facteurs peuvent être ajoutés pendant les premiers passages. Par exemple jusqu'au passage 3 ou 10, on peut ajouter dans le milieu du bFGF à une concentration finale de 1 ng/ml et de l'IL-11 à une concentration finale de 1 ng/ml.

L'ensemencement est réalisé dans ce milieu sur le "feeder" inactivé composé de fibroblastes de souris établies en lignée, les cellules STO. Dans certains cas, ces cellules ont été transfectées avec des vecteurs d'expression simples permettant l'expression de facteurs de croissance comme le SCF aviaire, de façon constitutive dans les cellules STO. Ainsi, ce 'feeder' produit le facteur sous une forme soluble et/ou attachée dans la membrane plasmique des cellules.

Après l'ensemencement initial des cellules directement dans ce milieu, le milieu est changé partiellement le lendemain, puis partiellement ou totalement durant les jours suivants, selon la vitesse d'adhésion observée des cellules primaires. Après environ 4 à 7 jours selon les cas, la culture initiale est dissociée et passée dans de nouvelles boîtes dans le même milieu initial sur le feeder inactivé. Après trois à cinq passages, les cellules sont cultivées sur un feeder inactivé de cellules STO non transfectées ou transfectées avec un vecteur d'expression codant pour une résistance à un antibiotique comme le gène de résistance à la néomycine, à l'hygromycine, à la puromycine .... . Après une vingtaine de passages, les cellules sont progressivement sevrées en facteurs de croissance et cytokines. Par sevrage progressif, on entend une suppression facteur par facteur dans le milieu de culture. Ainsi à un passage, on enlève d'abord le SCF, puis, deux ou trois passages après, l'IGF-1. Si les cellules ne présentent pas d'altérations morphologiques ou de variation dans leur vitesse de prolifération moyenne, les autres facteurs, comme le CNTF, l'IL-6 sont ensuite supprimés. Ce sevrage peut être également drastique. On enlève alors en une seule fois tous les facteurs. Les cellules sont alors observées et ne sont passées que plusieurs jours après si leur vitesse de prolifération est modifiée. Cette dernière solution est en général celle qui est pratiquée.

Différents isolats sont ainsi obtenus et maintenus pendant des périodes de temps très longues. Par périodes de temps très longues, on entend des durées de l'ordre de plusieurs semaines avec un minimum de 50 jours, de préférence des durées supérieures à 200 à 400 jours, sans limitations dans le temps. Des durées supérieures à 600 jours sont observées.

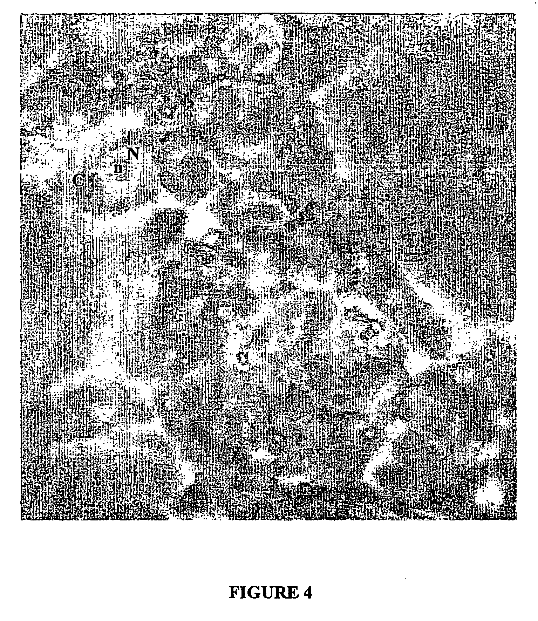

Quel que soit le support utilisé toutes les cellules qui sont adhérentes sont dissociées avec une enzyme protéolytique de dissociation, telle que la pronase, la collagénase, la dispase, la trypsine, etc.... De préférence, un enzyme protéolytique d'origine bactérienne est utilisé, afin d'éviter tout contaminant potentiel d'origine animale. Ces cellules ont les caractéristiques des cellules souches embryonnaires avec une morphologie spécifique illustrée, à titre d'exemple, par la **photo de la** **Figure 4** i.e. une petite taille, un rapport nucléocytoplasmique important, un noyau avec au moins un nucléole bien visible et un très faible cytoplasme. Ces cellules sont caractérisées par une croissance en massifs plus ou moins compacts. Les cellules adhérentes et non adhérentes présentent une réactivité croisée avec un certain nombre d'anticorps, comme précédemment décrit dans Pain et al., 1996 et dans les brevets US 6,114,168 et EP 787 180. La composante activité télomérase endogène est également présente et est un élément important de la nature 'souche' de ces cellules.

Des cellules de différents isolats sont obtenues et maintenues pendant de longue période de temps.

**Le tableau 1** illustre quelques unes des caractéristiques de ces isolats.

| ***Nom*** | **Espèce** | **Début** | **« Arrêt »** | **jours** | **Passage** | **Génération** |
|---|---|---|---|---|---|---|
| | | | | | | |
| S86N 16 | Poulet S86N | 26-01-2000 | 05-08-2001 | 559 | 207 | 692 |
| WL3 | Poulet WL | 28-06-2000 | 09-08-2001 | 403 | 153 | 333 |
| Valo4 | Poulet Valo | 26-09-2000 | 07-02-2002 | 401 | 135 | 317 |
| S86N 45 | Poulet S86N | 29-01-2001 | 12-11-2001 | 287 | 118 | 329 |

On notera que le terme « arrêt » ne correspond pas à la fin de la prolifération des cellules mais à un arrêt volontaire des cultures de cellules de la part de l'expérimentateur. Le nombre de génération n est obtenu par la formule X= 2 ⁿ ou X est le nombre cumulé théorique de cellules. Ce nombre est disponible puisque les cellules sont comptées à chaque passage et lors de chaque ensemencement. L'historique complet de la culture est ainsi disponible.

### Exemple 3 : Passage des cellules

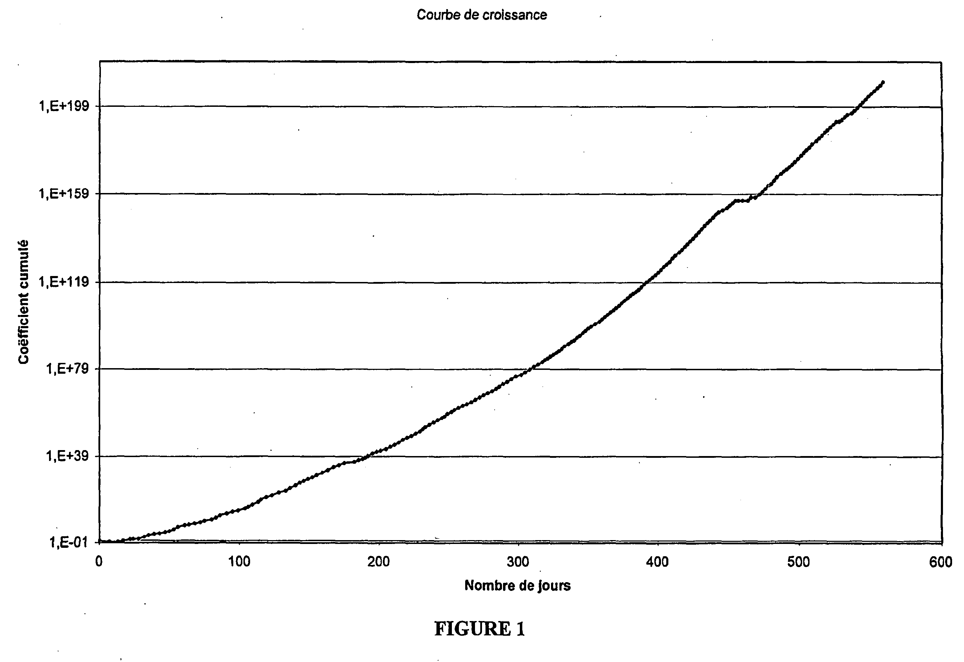

Une des caractéristiques des cellules souches , notamment des souches somatiques et des cellules souches embryonnaires, est leur aptitude à proliférer *in vitro* pendant des périodes de temps considérables. Afin de propager et de passer les cellules, le milieu de culture est changé et remplacé par du milieu neuf quelques heures avant leur passage. La Courbe présentée en **figure 1** illustre un profil de croissance et d'établissement des cellules.

### Exemple 4 : temps de doublement et temps moyen de division

A partir des cellules établies en culture et présentées dans les exemples précédents, un temps moyen de division peut être calculé. Pour l'ensemble des isolats indépendants obtenus, la vitesse de prolifération s'accentue légèrement au cours des passages successifs faisant ainsi évoluer le temps moyen de division au cours de l'établissement des cellules. En phase adhérente, les cellules sont initialement ensemencées sur un tapis nourricier inactivé (appelé 'feeder') et sont passées régulièrement à une densité constante d'ensemencement initial de 1 à 2 x 10⁶ cellules par boîte de 100 mm. Le tableau 2 illustre le temps de doublement (d) et le temps moyen de division (TMD en heure) pour 3 types cellulaires établis en fonction du temps de culture. On observe que le temps moyen de doublement diminue au cours de l'établissement.

**Tableau 2 :**

| Cellules/Jours | 50 | 100 | 150 | 200 | 250 | 300 | 350 | 400 | 450 | 500 | 550 |
|---|---|---|---|---|---|---|---|---|---|---|---|
| | | | | | | | | | | | |
| S86N 16 (d) | 0,30 | 0,63 | 1,00 | 0,86 | 1,13 | 1,15 | 1,47 | 1,70 | 1,94 | 1,50 | 1,9. |
| S86N 16 (TMD) | 80 | 38 | 24 | 27,9 | 21,2 | 20,9 | 16,3 | 14,1 | 12,4 | 16 | 12,6 |
| S86N 45 (d) | 0,49 | 0,89 | 0,89 | 1,45 | 2,15 | x | x | x | x | x | x |
| S86N45 (TMD) | 49 | 26,8 | 27 | 16,5 | 11,1 | x | x | x | x | x | x |
| Valo 4 (d) | 0,03 | 0,61 | 1,00 | 1,17 | 1,26 | 1,03* | 1,08* | 1,25* | x | x | x |
| Valo 4 (TMD) | >48 | 39,3 | 24 | 20,5 | 19 | 23,3 | 22,2 | 19,2 | x | x | x |

Le temps de doublement moyen d est établi pour la période de temps indiquée en jours avec la formule suivante : d = (1/Log2 x (LogX2/X1))x 1/(T2-T1) où X2 et X1 sont les nombres totaux de cellules aux instants T2 et T1. Cette formule est la conséquence directe du calcul du nombre de génération N par la formule X= 2 "présenté dans l'exemple 1. Le Temps moyen de division (TMD) est alors obtenu en heures en divisant 24 heures par d.
* les cellules Valo sont passées au cours de cet établissement sur support plastique sans présence de feeder. Le temps de doublement diminue puis re-augmente, quand les cellules se réhabituent à ce nouvel environnement.

### Exemple 5 : Contrôle du niveau de sérum pour la prolifération des lignées

Lors de l'obtention de ces lignées, les milieux de culture utilisés sont des milieux de culture classiques comprenant une base (DMEM, GMEM, HamF12, McCoy, etc...) supplémentée de différents additifs tels que des acides aminés non essentiels, des vitamines, du pyruvate de sodium. Ce milieu complexe comprend du sérum de veau foetal, qui demeure un élément central de la culture, même si des composants de différentes origines dont des composants végétaux peuvent être progressivement utilisés. Un processus pour contrôler et habituer les cellules à des proportions relativement faibles de sérum de veau foetal est présenté. On parvient ainsi à maintenir des cellules en prolifération importante (Temps de division > 1) avec des pourcentages de sérum faible (2% par exemple dans le cas des cellules S86N 16).

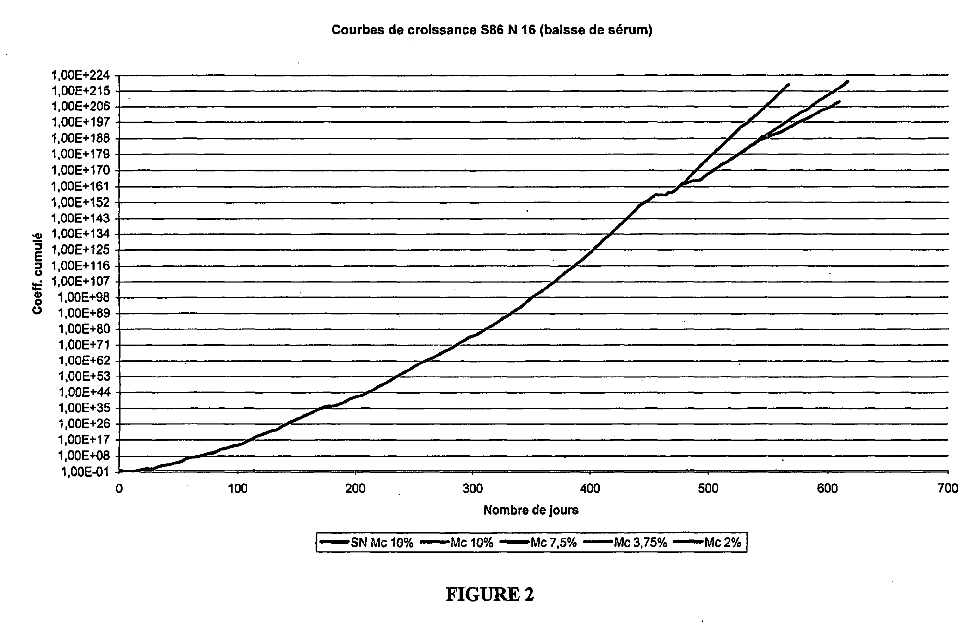

Les courbes présentées en **figure 2** illustre la diminution relative de sérum pour un type cellulaire donné : les cellules S86N 16. Le temps de doublement et les temps moyens de division ont été également calculés et portés dans le **tableau 3**. On notera que le temps moyen de division augmente en fonction de la diminution relative de sérum. Une phase de rattrapage est néanmoins observée après quelques temps en culture dans les conditions mentionnées. Ce temps reste néanmoins inférieure à 24h (d>1), ce qui représente déjà une prolifération très intéressante en terme industriel même à des concentrations de sérum de 2 %, ce qui est déjà relativement faible. Des améliorations quant aux différents métabolites à utiliser peuvent être envisagées pour accroître ce temps et encore optimiser davantage les conditions de culture.

**Tableau 3 :**

| Condition | 10% | 7,5% | 3,75% | 2% |
|---|---|---|---|---|
| d | 2,02 | 1,51 | 1,47 | 1,08 |
| TMD | 11,9 | 15,8 | 16,3 | 22,2 |

Les exemples sont pris entre les passages p204 et p179 pour la condition 10%, entre p198 et p176 pour le 7,5%, entre p224 et p201 pour le 3,75 % et entre p216 et p199 pour le 2%.

### Exemple 6 : Sevrage des cellules en tapis nourricier

Dans les conditions initiales de culture, la présence d'un tapis de cellules inactivées semble nécessaire pour obtenir des cellules souches embryonnaires comme il a été décrit préalablement. Ce tapis nourricier ou 'feeder' ne semble plus nécessaire après un certain nombre de passages. Seul le plastique 'traité culture' apparaît important. En effet, une des caractéristiques de certaines cellules eucaryotes est de proliférer sous forme adhérente. Afin de faciliter l'adhésion des cellules, les différents matériels plastiques utilisés sont traités 'culture'. Ils subissent lors de leur fabrication un traitement qui ajoute des charges à la surface du plastique, charges qui favorisent les adhésions de la matrice extracellulaire des cellules. A l'inverse, le plastique non traité culture cellulaire, souvent appelé plastique de qualité bactériologique, n'est pas traité en surface par addition de charges spécifiques. L'adhésion des cellules y est en général très difficile, voir impossible, ou alors induit des changements de morphologie, de comportement souvent drastiques. Cette distinction entre les deux qualités de plastique permet d'obtenir selon les ensemencements qui y sont effectués des cellules avec des comportement différents. Un sevrage progressif des cultures en 'feeder' inactivé permet d'obtenir après quelques passages des cultures homogènes de cellules souches directement ensemencées sur plastique 'traité culture'.

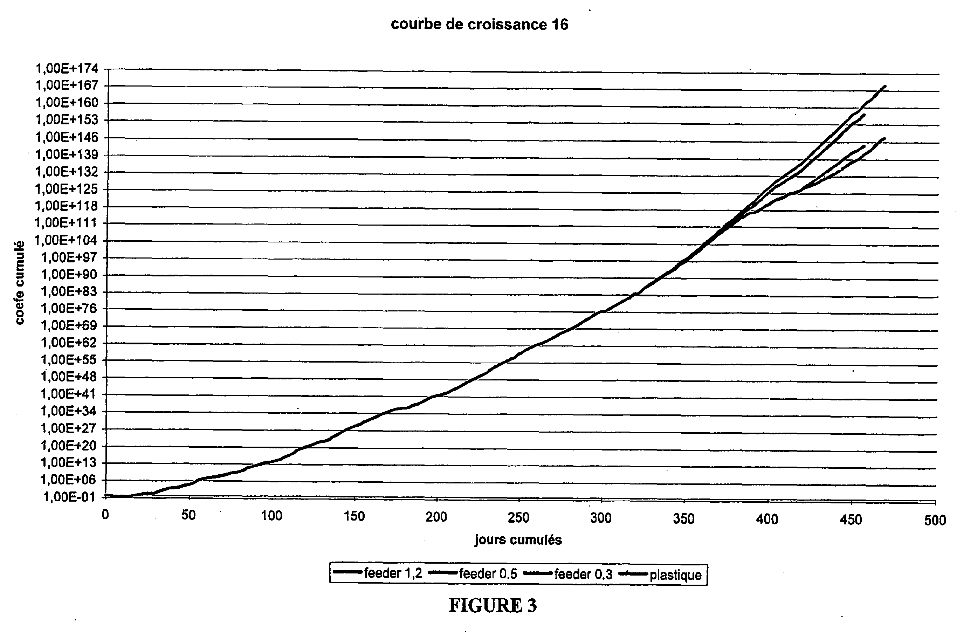

Les courbes de croissance comparative des cellules maintenues en présence et en absence de 'feeder' inactivé sont présentées avec le cas des cellules S86N 16 en **figure 3**. Cette adaptation des cellules est progressive afin de ne pas perdre le caractère de cellules souches des cellules initialement maintenues sur 'feeder'. Des dérives progressives sont ainsi faites. L'obtention de cellules proliférant sur plastique est l'aboutissement du processus de sevrage. Dans le **tableau 4,** les temps de division montrent une sensibilité des cellules à leur environnement. Comme dans le cas du sevrage progressif en sérum, une adaptation est obtenue avec un effet de rattrapage sur les cellules après quelques passages dans les conditions définies.

**Tableau 4 :**

| Condition | 1,2 | 0,5 | 0,3 | plastique |
|---|---|---|---|---|
| d | 1,95 | 1,84 | 1,39 | 1,42 |
| TMD | 12,3 | 13 | 17,3 | 16,9 |

Les exemples sont pris entre les passages p154 et p131 pour les 3 conditions 1,2 x 10⁶, 0,5 x 10⁶ et 0,3 x 10⁶ cellules feeder et entre p161 et p139 pour la condition sur plastique seul.

### Exemple 7 : Sevrage des cellules en facteurs de croissance

Dans les conditions initiales de culture, la présence de facteurs de croissance est nécessaire. On peut distinguer schématiquement deux familles de facteurs : les cytokines et les facteurs trophiques.

Les cytokines sont principalement des cytokines dont l'action passe par un récepteur qui s'associe à la protéine gp130. Ainsi, le LIF, l'interleukine 11, l'interleukine 6, le CNTF, l'oncostatine, la cardiotrophine, ont un mode d'action semblable avec le recrutement au niveau du récepteur d'une chaîne spécifique et de l'association de cette dernière avec la protéine gp130 sous forme monomérique ou parfois hétérodimérique. Dans quelques cas, l'association d'une forme soluble des récepteurs, forme décrite entre autres pour les récepteurs de l'interleukine 6 et du CNTF, permet d'accroître l'effet prolifératif observé. Il a été montré préalablement que l'addition d'au moins une de ces cytokines semblait nécessaire à l'obtention des cellules souches embryonnaires.

Les facteurs trophiques sont principalement le SCF, l'IGF-1, le bFGF, qui sont également utilisés au départ de la culture, comme décrit précédemment. Leur présence est également nécessaire pour obtenir et amplifier les cellules.

En diminuant progressivement ces facteurs de croissance, il est possible d'obtenir après quelques passages des conditions de culture qui permettent la prolifération des cellules souches embryonnaires ou somatiques sans l'addition de facteur de croissance exogène. Les différents marqueurs utilisés pour caractériser ces cellules sont toujours positifs pour les cellules maintenues sans facteurs.

### Exemple 8 : Comparaison des milieux utilisés

Ensemencées dans des milieux différents, les cellules ne sont pas obtenues avec les mêmes fréquences. La comparaison des compositions des milieux rend difficile l'identification d'un des composants en particulier. Il semble plus vraisemblable que l'ensemble de l'association permette un mieux dans la physiologie des cellules. Parmi les milieux privilégiés, on notera le milieu Ham F12, MacCoy, DMEM et un milieu DMEM enrichi en biotine. A partir d'un même isolat, des essais d'adaptation dans ces différents milieux sont réalisés.

### Exemple 9 : Etablissement des cellules non adhérentes

Lors des passages successifs des cellules souches, un ensemencement à forte densité directement en boîte bactériologique permet d'obtenir après quelques passages des cellules embryonnaires qui se détachent de leur substrat et qui prolifèrent en suspension sous forme de petits agrégats réguliers. Cette prolifération est encouragée sur plusieurs passages par simple dilution, dissociation mécanique et non-usage d'enzyme protéolytique. L'agitation des cultures est en général pratiquée mais ne représente pas un élément discriminant pour l'obtention des cellules non adhérentes. Comme les cellules adhérentes, ces cellules présentent une morphologie caractéristique de cellules souches i.e. une petite taille, un rapport nucléocytoplasmique important, un noyau avec au moins un nucléole bien visible et un très faible cytoplasme. Ces cellules sont caractérisées par une croissance en petits agrégats plus ou moins compacts. Ces cellules non adhérentes présentent une réactivité croisée avec un certain nombre d'anticorps, comme précédemment décrit dans Pain et al., 1996. Ces cellules sont également positives pour l'activité télomérase endogène (comme présenté dans l'exemple 10 pour les cellules EB1, EB4 et EB5). En phase non adhérente, les cellules présentent une prolifération importante dans différents milieux. La densité initiale d'ensemencement ainsi que l'approvisionnement en milieu frais de façon très régulière assure des densités importantes qui peuvent aller au delà de 1 x 10 ⁶ cellules par ml. Le **tableau 5** résume les principales caractéristiques de quelques isolats (cellules parentales, passage initial de la mise en suspension, nombre de jours maintenus en culture en suspension, nombre de passages et de générations obtenues avant un arrêt volontaire des entretiens). On peut ainsi remarquer que le passage pour la mise en suspension peut varier d'un isolat à l'autre (Voir isolat EB1 et EB14) ainsi que la vitesse de prolifération (voir isolat EB3 et EB14).

**Tableau 5 :**

| Nom | Cellules parentales | Passage initial | Début | jours | Passages | Générations |
|---|---|---|---|---|---|---|
| EB1 | S86N 16 | p111 | 20-01-2001 | 184 | 41 | 120 |
| EB3 | S86N 16 | p118 | 23-01-2001 | 381 | 17 | 40 |
| EB4 | S86N 45 | p100 | 25-09-2001 | 44 | 17 | 40 |
| EB5 | S86N 45 | p100 | 25-09-2001 | 44 | 17 | 40 |
| EB14 | S86N 45 | p81 | 5-09-2002 | 70 | 24 | 65 |

On notera que le terme « Début » correspond à la mise en non-adhérence des cellules.

### Exemple 10 : Caractérisation des cellules établies

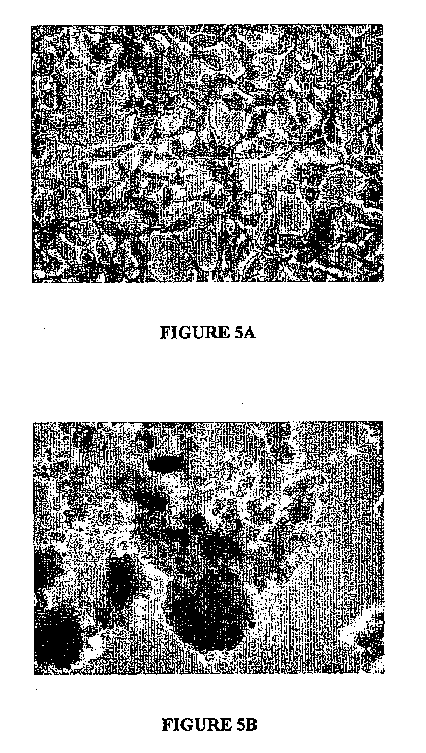

Les cellules souches maintenues pendant des temps longs de culture sont caractérisées avec les mêmes critères que ceux décrits précédemment (Pain et al., 1996). On peut ainsi détecter de façon régulière l'activité alcaline phosphatase endogène, illustrée par la photo de la figure 5, l'activité télomérase endogène et la réactivité avec certains anticorps spécifiques comme les anticorps SSEA-1 (TEC-01) et EMA-1.

Un des critères important lors de l'établissement des cellules est la présence de la télomérase. Différents tests ont été faits au cours du maintien en culture des cellules à l'aide d'un kit de détection de la TRAP (Telomerase PCR Elisa, Roche). Les cellules sont détectées positives après différents passage en culture. Ainsi, l'activité télomérase est détectable pour les cellules S86N 16, les cellules S86N 45 ainsi que pour les cellules EB1, EB4 et EB5 qui en sont dérivées sous une forme non adhérente (voir **tableau 6**). Les CEF (Chicken Embryonic Fibroblasts) maintenus en culture primaire sont considérés comme négatifs. Le seuil d'une D.O. < 0,2 est le seuil recommandé par le kit comme le seuil négatif. Toutes les analyses ont été réalisées sur un équivalent de 2000 cellules.

**Tableau 6 : Dosage de l'activité télomérase dans différentes lignées à différents passages**

| Cellules | Passage | Télomérase D.O. |
|---|---|---|
| S86N 16 | p12 | 1,7 |
| | p29 | 2,8 |
| | p185 | 0,97 |
| | p204 | 0,95 |
| S86N 16 EB1 | p134 | 1, 1 |
| S86N 45 | p50 | 0,87 |
| | p58 | 1,1 |
| | p66 | 0,96 |
| | p94 | 1,2 |
| S86N 45 EB 4 | p112 | 1,4 |
| S86N 45 EB5 | p112 | 0,94 |
| CEF * | p4 | 0,07 |
| | | |

### Exemple 11 : Transfection et induction des cellules

Les cellules souches maintenues en croissance sur le long terme sont transfectées avec différents plasmides d'expression. Il a été montré que les cellules souches aviaires pouvaient être transfectées (Pain et al., 1999). En particulier, les cellules non adhérentes sont transfectées et différents systèmes de tri permettent de repérer les cellules transfectées de façon stable (tri cellulaire, dilution limite, ...). Ces modifications génétiques peuvent s'effectuer au stade non différencié de la cellule souche. Cette modification obtenue, la cellule est alors induite à différencier spontanément ou par addition d'inducteur de différenciation. Dans ce cas, on peut utiliser l'acide rétinoïque à des concentrations de 10⁻⁸ M à 10⁻⁶ M, ou le dimethysulfoxide à des concentrations de 1 à 2 % final ou le butyrate de sodium à des concentrations de 10⁻⁴ à 10⁻⁸ M, ou le phorbol ester (TPA, PMA, ...) ou encore les lipopolysaccharrides (LPS) à des concentrations de 1 à 5 ug /ml final. Dans un autre exemple, les cellules peuvent former des corps embryoïdes en suspension, corps embryoïdes qui peuvent être mis à adhérer sur plastique après dissociation ou non des cellules qui les constituent. Ces cellules différenciées prolifèrent alors mais ont une capacité plus limitée de prolifération dans le long terme. En ciblant la modification génétique sur un gène qui influence la prolifération des cellules, on peut rendre ces cellules différenciées capables de proliférer sur le long terme.

### Exemple 12 : Infection des cellules

Les cellules adhérentes et non adhérentes sont infectables par différents virus et rétrovirus dont les virus et rétrovirus aviaires. Ces cellules peuvent ainsi servir de support de réplication pour la production de stocks viraux destinés à la production de vaccins humains et vétérinaires vivants, atténués ou inactivés selon les cas. Parmi les virus d'intérêt, on peut citer ceux de la famille des adénovirus (comme Human Adenovirus C, Fowl Adenovirus A, Ovine adenovirus D, Turkey Adenovirus B), des circoviridés (comme Chicken Anemia Virus, CAV), certains coronavirus, comme le virus de la bronchite infectieuse aviaire (IBV), les flavivirus (comme Yellow fever virus et hepatitis C virus), les hepadnavirus (comme Hepatitis B virus et Avihepadnavirus tel que Duck hepatitis B virus); les herpesvirus (comme les Gallid herpesvirus, HSV (Herpes simplex virus) et Human herpesvirus 1, 3 et 5), les orthomyxovirus (comme le virus de la grippe : Influenzavirus A, Influenzavirus B et Influenzavirus C), les papovavirus (comme polyomavirus et plus particulièrement Simian virus 40), les paramyxovirus (comme les virus de la rougeole, des oreillons, de la rubéole et comme les respirovirus et pneumovirus tel que Human respiratory syncytial virus et Metapneumovirus tel que Avian pneumovirus), les picornavirus (comme le virus de la polio, de l'hépatite A, et tel que Encephalomyocarditis virus et Foot-and-mouth disease virus), les poxvirus, (comme les fowlpox virus et les avipox dont les canaripox, les Juncopox virus, les Mynahpox virus, les Pigeonpox virus, les Psittacinepox virus, les Quailpox virus, les Sparrowpox virus, les Starlingpox virus, les Turkeypox virus), les reovirus (comme les rotavirus), les rétrovirus (comme les ALV, avian leukosis virus, les Gammaretrovirus tel que Murine leukeamia virus, les Lentivirus tel que Human immunodeficiency virus 1 et 2) et les Togaviridae tel que Rubivirus, notamment Rubella virus.

### Exemple 13: protocole d'infection d'une lignée cellulaire aviaire non adhérente (EB1) par un virus

### Amplification des cellules :

Les cellules EB1 ou EB14 sont ensemencées dans un milieu, de préférence du MacCoy's 5A, du HAMF12, du DMEM, ou tout autre milieu intéressant, contenant 5 % de serum à une concentration de 0.2 10⁶ cellules/ml pour un volume initial de 50 ml en général. Elles sont maintenues en culture à 39°c et à 7.5 % de CO2, sous agitation. Du milieu neuf est ajouté chaque jour pendant les 3 à 4 jours que durent l'amplification pour atteindre une concentration cellulaire de 1 à 3 x 10 ⁶ cellules /ml pour un volume final de culture de 100 à 250 ml.
Les cellules en suspension sont prélevées et centrifugées pendant 10 min à 1000 rpm environ. Le culot est resuspendu dans 20 à 50 ml de PBS 1X (Phosphate buffer Salt). Les cellules sont alors comptées, centrifugées et les cellules en culot sont reprises dans un milieu sans sérum à une concentration finale de 3 à 5.10⁶ cellules/ml. Plusieurs tubes sont alors préparés dans ces conditions contenant de 3 à 5 x 10⁶ cellules par tube.

### Préparation du virus et infection :

Le stock viral de titre connu est décongelé rapidement à 37°c et dilué dans du milieu sans sérum à un titre de 10x à 1000 x la concentration nécessaire à l'infection finale. Les cellules sont infectées par le virus d'intérêt à une m.o.i. (multiplicity of infection) de 0.01 à 0.5 selon les types de virus, ce qui fait ajouter entre 0,1 et 10% volume/volume de suspension virale au culot cellulaire. Après 1 heure d'incubation à la température optimale pour le virus, en général de 33 à 37°c, les cellules sont centrifugées à nouveau et le milieu enlevé avec précaution. Cette étape s'avère souvent nécessaire pour limiter l'effet du virus initial dans le processus ultérieur. Une des possibilités est de diluer directement les cellules sans les centrifuger à nouveau avec du milieu avec sérum (5% de sérum) à une concentration finale de 0,2 à 1.10⁶ cellules/ml et remises en incubation.

### Récolte du surnageant et des cellules :

Après 2 à 4 jours d'incubation, selon les cinétiques virales et l'effet cytopathique éventuel de certains virus, le milieu contenant les cellules ou les débris cellulaires est récolté. Selon les virus, seuls le culot ou le surnageant peuvent être intéressants et contenir les particules virales. Les cellules sont récoltées et centrifugées. Le surnageant recueilli est centrifugé de nouveau 5 à 10 minutes à 2500 rpm, et conservé à -80°c avant la purification des particules. Un aliquot est prélevé pour la réalisation du titrage. Le culot cellulaire est repris dans 5 ml de milieu sans serum, soniqué, et centrifugé 5 à 10 minutes à 2500 rpm. Le surnageant obtenu est conservé à-80°c jusqu'à la purification et le titrage d'un aliquot.
Les efficacités d'infection et de production virales sont comparées entre les différentes conditions réalisées.
Pour les virus à effets cytopathiques, les titrages sont en général réalisés par la technique des plages de lyse.

### Exemple 14: protocole d'infection d'une lignée cellulaire aviaire adhérente (S86N 45) par un virus

### Préparation des cellules :

Les cellules sont ensemencées 48 heures avant l'infection dans des flacons T150 à une concentration comprise entre 0.03 et 0.06 10⁶ cellules/cm2 dans un milieu, de préférence du milieu MacCoy's 5A, du HAMF12, du DMEM, ou tout autre milieu intéressant, contenant 5% de sérum. Elles sont maintenues à 39°C et 7.5% CO2.

### Infection :

Le stock viral de titre connu est décongelé rapidement à 37°c et dilué dans du milieu sans sérum à un titre de 10x à 1000 x la concentration nécessaire à l'infection finale. Les cellules sont infectées par le virus d'intérêt à une m.o.i. (multiplicity of infection) de 0.01 à 0.5 selon les types de virus, ce qui fait ajouter entre 0,1 et 10% volume/volume de suspension virale au culot cellulaire. L'infection est en général réalisée dans un minimum de milieu (de 5 à 10 ml pour un flacon de 75 cm²) dans un milieu à 0% sérum.

Après 1 heure d'incubation à la température optimale pour le virus, en général de 33 à 37°c, 20 ml de milieu 5 % sont rajoutés dans les flacons. Dans un cas particulier, les cellules peuvent être lavées avec du PBS pour éliminer les particules qui ne seraient pas attachées aux cellules. Dans le cas d'un virus cytopathique, les cellules sont observées quotidiennement après l'infection pour suivre l'apparition de plage de lyse cellulaire, indicatrice du bon déroulement de l'infection.

### Récolte du surnageant et des cellules :

Après 2 à 4 jours d'incubation selon les cinétiques virales et l'effet cytopathique éventuel de certains virus, le milieu contenant le surnageant, les cellules et les débris cellulaires sont récoltés. Selon les virus, seuls le culot ou le surnageant peuvent être intéressants et contenir les particules virales. Les cellules sont récoltées et centrifugées. Le surnageant recueilli est centrifugé de nouveau 5 à 10 minutes à 2500 rpm, et conservé à -80°c avant la purification des particules. Un aliquot est prélevé pour la réalisation du titrage. Le culot cellulaire est repris dans 5 ml de milieu sans serum, soniqué, et centrifugé 5 à 10 minutes à 2500 rpm. Le surnageant obtenu est conservé à -80°c jusqu'à la purification et le titrage d'un aliquot.
Les efficacités d'infection et de production virales sont comparées entre les différentes conditions réalisées.
Pour les virus à effet cytopathique, les titrages sont en général réalisés par la technique des plages de lyse.

### Exemple 15: Réplication d'un avipox recombinant sur les cellules souches aviaires non adhérentes de la lignée EB1

Les cellules souches non adhérentes EB1 à passage 138 sont amplifiées puis infectées à une m.o.i de 0,1 par un avipox recombinant produisant une protéine d'intérêt.
Après l'infection, les cellules sont maintenues en spinner pendant les 4 jours que dure l'infection. Un aliquot est prélevé à partir du deuxième jour puis les deux jours suivants pour suivre l'évolution du titre viral à la fois au niveau du surnageant et au niveau du contenu intracellulaire, après lyse du contenu cellulaire. La titration a été réalisée par la technique des plages de lyse.
Le **tableau 7** illustre les résultats obtenus. Ces résultats mettent en évidence la réplication très satisfaisante de l'avipox recombinant sur les cellules souches EB 1. Ainsi le titre infectieux progresse tout au long de la culture et du déroulement de l'infection pour atteindre un maximum après 4 jours d'incubation de 7.2 PFU/cellule (PFU : Plating Forming Unit). Ce titre est au moins équivalent à celui obtenu pour ce même avipox recombinant sur des cellules primaires d'embryons de poulet.
Ce titre est susceptible d'être amélioré par des conditions particulières de culture et des processus d'optimisation.
On notera également que des titres infectieux au moins équivalents ont également été obtenus à plus grande échelle dans des bioréacteurs de 3 litres.

**Tableau 7 : Cinétique de titrage de l'avipox recombinant sur les cellules souches EB1 non adhérentes**

| Prélèvement (h après infection) | **50 heures** | **74 Heures** | **97 Heures** |
|---|---|---|---|
| **fraction cellulaire** (Log PFU/ml) | 6.40 | 6.37 | 5.99 |
| **Surnageant** (Log PFU/ml) | 5.56 | 5.8 | 6.29 |
| **Total** (Log PFU/ml) | 5.78 | 5.94 | 6.31 |
| **PFU/cellule** | 2.2 | 3.2 | 7.2 |

### REFERENCES

Baba TW, Humphries EH. (1985). Formation of a transformed follicle is necessary but not sufficient for development of an avian leukosis virus-induced lymphoma. Proc Natl Acad Sci U S A 82 :213-216.
Beug H, von Kirchbach A, Doderlein G, Conscience JF, Graf T. (1979). Chicken hematopoietic cells transformed by seven strains of defective avian leukemia viruses display three distinct phenotypes of differentiation. Cell 18 : 375-390.
Guilhot C, Benchaibi M, Flechon JE, Samarut J. (1993). The 12S adenoviral E1A protein immortalizes avian cells and interacts with the avian RB product. Oncogene 8 : 619-624
Kawaguchi T, Nomura K, Hirayama Y, Kitagawa T. (1987). Establishment and characterization of a chicken hepatocellular carcinoma cell line, LMH. Cancer Res 1987 47 : 4460-4464.
Kim H, You S, Farris J, Foster LK, Foster DN. (2001). Post-transcriptional inactivation of p53 in immortalized chicken embryo fibroblast cells. Oncogene 20 :3306-3310.
Kim H, You S, Kim IJ, Foster LK, Farris J, Ambady S, Ponce de Leon FA, Foster DN. (2001). Alterations in p53 and E2F-1 function common to immortalized chicken embryo fibroblasts. Oncogene 20 :2671-2682.
Liu JL, Klein PA, Moscovici MG, Moscovici C. (1992). Monoclonal antibodies recognizing normal and retrovirus-transformed chicken hematopoietic cells. Virology 189 : 583-591.
Moscovici C, Moscovici MG, Jimenez H, Lai MM, Hayman MJ, Vogt PK. (1977). Continuous tissue culture cell lines derived from chemically induced tumors of Japanese quail. Cell 11 : 95-103.
Pain B., Clark M.E., Shen M., Nakazawa H., Sakurai M., Samarut J., Etches RJ (1996). Long-term in vitro culture and characterisation of avian embryonic stem cells with multiple morphogenetic potentialities. Development 122 : 2339-2348.
Pain B., Chenevier P., Samarut J. (1999). Chicken embryonic stem cells and transgenic strategies. Cells Tissues Organs 165 : 212-219.
Samarut J, Gazzolo L. (1982). Target cells infected by avian erythroblastosis virus differentiate and become transformed. Cell 28 : 921-929.
Smith JR and Pereira-Smith OM (1996). Replicative senescence: implications for in vivo aging and tumor suppression. Science 273, 63-67.

## Revendications

1. Procédé d'obtention de lignées cellulaires aviaires, **caractérisé en ce qu'**il comprend les étapes suivantes :
a) mise en culture de cellules souches embryonnaires aviaires dans un milieu comportant :
- au moins les facteurs de croissance SCF, IGF-1 et bFGF et au moins une cytokine choisie parmi le LIF, l'IL-11, l'IL-6, l'IL-6R, le CNTF, l'oncostatine et la cardiotrophine;
- un tapis nourricier inactivé de cellules STO ; et
- du sérum de veau foetal à une concentration de 12 à 8 %,
b) après une vingtaine de passages, le milieu de culture est modifié :
- par sevrage progressif desdits facteurs de croissance et de ladite cytokine ; ou
- par sevrage progressif du tapis nourricier ; ou
- par diminution de la concentration en sérum de veau foetal jusqu'à arriver à des faibles proportions de 2 %,
c) établissement de lignées cellulaires adhérentes ou non-adhérentes capables de proliférer dans un milieu de base en l'absence de facteurs de croissance exogènes et de cytokine, de sérum ou du tapis nourricier inactivé, l'établissement de lignées cellulaires non-adhérentes étant obtenu par ensemencement par au moins 1 x 10⁶ cellules/ml en boîte bactériologique suivi de plusieurs passages en dilution simple.

2. Procédé selon la revendication 1, **caractérisé en ce que** les cellules issues des lignées obtenues à l'étape c) sont capables de proliférer pendant au moins 50 jours, de préférence au moins 600 jours.

3. Procédé selon la revendication 1 ou 2, **caractérisé en ce que** les cellules obtenues à l'étape c) sont soumises à une sélection dans des milieux de cultures utilisés pour la production à grande échelle pour obtenir des clones adaptés à la production de vaccin destiné à la thérapie humaine ou animale.

4. Procédé selon l'une des revendications 1 à 3, **caractérisé en ce que** les cellules issues des lignées obtenues à l'étape c) sont des cellules souches aviaires.

5. Procédé selon l'une des revendications 1 à 3, **caractérisé en ce que** les cellules issues des lignées obtenues à l'étape c) sont des cellules embryonnaires souches aviaires.

6. Procédé selon l'une des revendications 1 à 5, **caractérisé en ce qu'**il comprend à la fin de l'étape c) une étape de prolifération des cellules souches adhérentes en l'absence du tapis nourricier inactivé.

7. Procédé selon l'une des revendications 1 à 5, **caractérisé en ce qu'**il comprend à la fin de l'étape c) une étape de prolifération des cellules souches non-adhérentes issues des lignées obtenues à l'étape c) en suspension.

8. Procédé selon l'une des revendications 1 à 5, **caractérisé en ce qu'**il comprend à la fin de l'étape c) une étape de prolifération des cellules souches non-adhérentes issues des lignées obtenues à l'étape c) en suspension dans un milieu dépourvu de facteurs de croissance exogènes et de cytokine.

9. Procédé selon l'une des revendications précédentes 1 à 8, **caractérisé en ce que** les cellules issues des lignées obtenues à l'étape c) prolifèrent dans un milieu en sérum foetal à des proportions de 2 %.

10. Procédé selon l'une des revendications1 à 9, **caractérisé en ce que** les cellules issues des lignées obtenues à l'étape c) présentent au moins une des caractéristiques suivantes :
- un nucléole bien visible et un très faible nucléoplasme,
- une activité alcaline phosphatase endogène,
- une activité télomérase endogène,
- une réactivité avec des anticorps spécifiques sélectionnés parmi le groupe des anticorps SSEA-1 (TEC01), SSEA-3 et EMA-1.

11. Procédé selon l'une des revendications 1 à 10, **caractérisé en ce que** les cellules utilisées à l'étape a) sont des cellules obtenues par suspension de cellules provenant de disques blastodermiques d'oeufs fécondés dans un milieu de culture comprenant au moins une cytokine, b-FGF et SCF, lesdites cellules étant ensemencées sur un tapis de cellules nourricières, incubées, puis prélevées.

12. Procédé selon l'une des revendications 1 à 11, **caractérisé en ce que** l'étape b) consiste en un sevrage progressif de chaque facteur de croissance ajouté dans le milieu de l'étape a), comprenant un passage dans un milieu neuf dépourvu d'au moins un desdits facteurs et à répéter différents passages successifs jusqu'à ce que le milieu soit dépourvu de l'ensemble desdits facteurs.

13. Procédé selon l'une des revendications 1 à 12, **caractérisé en ce qu'**il comprend à la fin de l'étape c) une étape de prolifération des cellules issues des lignées obtenues à l'étape c) dans un milieu de base choisi parmi DMEM, GMEM, HamF12 ou McCoy supplémenté de différents additifs tels que des acides aminés non essentiels, des vitamines et du pyruvate de sodium.

14. Procédé selon l'une des revendications 1 à 13, **caractérisé en ce que** les cellules issues des lignées obtenues à l'étape c) sont modifiées génétiquement de sorte à produire une substance d'intérêt, notamment un polypeptide d'intérêt, un anticorps ou un virus atténué.

15. Procédé selon l'une des revendications 1 à 13, **caractérisé en ce que** les cellules issues des lignées obtenues à l'étape c) sont modifiées génétiquement de sorte qu'elles supportent la réplication de virus vivants ou atténués, en particulier les virus sélectionnés dans le groupe des adénovirus, des hepadnavirus, des herpesvirus, des orthomyxovirus, des papovavirus, des paramyxovirus, des picornavirus, des poxvirus, des réovirus et des rétrovirus.

16. Procédé selon la revendication 15, **caractérisé en ce que** les virus répliqués sur ces cellules appartiennent à la famille des orthomyxovirus, en particulier le virus de la grippe.

17. Procédé selon la revendication 15, **caractérisé en ce que** les virus répliqués appartiennent à la famille des paramyxovirus, en particulier les virus de la rougeole, des oreillons et de la rubéole.

18. Utilisation des lignées cellulaires ou cellules issues de ces lignées cellulaires obtenues par un procédé selon l'une des revendications 1 à 17, pour la production de substances d'intérêt, en particulier des protéines d'intérêt thérapeutiques, des anticorps.

19. Utilisation des lignées cellulaires ou cellules issues de ces lignées cellulaires obtenues par un procédé selon l'une des revendications 1 à 17, pour la réplication de virus vivants ou atténués, en particulier les virus pris dans le groupe des adénovirus, des circoviridés, des coronavirus, des flavivirus, des hepadnavirus, des herpesvirus, des orthomyxovirus, des papovavirus, des paramyxovirus, des picornavirus, des poxvirus en particulier des Avipoxvirus, notamment Fowlpox virus, Juncopox virus, Mynahpox virus, Pigeonpox virus, canarypox virus, Psittacinepox virus, Quailpox virus, Sparrowpox virus, Starlingpox virus et Turkeypox virus, des réovirus, des rétrovirus et des togaviridae.

20. Utilisation selon la revendication 19, pour la production de virus appartenant à la famille des orthomyxovirus, en particulier le virus de la grippe, notamment Influenza A, Influenza B et Influenza C.

21. Utilisation selon la revendication 19, pour la production de virus appartenant à la famille des paramyxovirus, en particulier les virus de la rougeole, des oreillons, de la rubéole, les respirovirus et pneumovirus .

22. Utilisation selon l'une des revendications 19 à 21, pour la production de vaccins humains ou vétérinaires vivants, atténués ou inactivés.

## Claims

1. A method for obtaining avian cell lines, **characterized in that** it comprises the following steps:
a) growing avian embryonic stem cells in a medium including:
- at least the growth factors SCF, IGF-1 and bFGF and at least one cytokine selected from LIF, IL-11, IL-6, IL-6R, CNTF, oncostatin and cardiotrophin;
- an inactivated feeder layer of STO cells; and
- fetal calf serum at a concentration of 12 to 8%
b) after about twenty passages, the culture medium is modified:
- by gradual withdrawal of said growth factors and of said cytokine; or
- by gradual withdrawal of the feeder layer; or
- by decreasing the fetal calf serum concentration until small proportions of 2% are reached,
c) establishing adherent or non-adherent cell lines capable of proliferating in a basic medium in the absence of exogenous growth factors and cytokine, serum or the inactivated feeder layer, the establishment of non-adherent cell lines being obtained by sowing at least 1 x 10⁶ cells/mL in a bacteriological dish followed by several passages with simple dilution.

2. The method according to claim 1, **characterized in that** the cells originating from lines obtained in step c) are capable of proliferating for at least 50 days, preferably at least 600 days.

3. The method according to claim 1 or 2, **characterized in that** the cells obtained in step c) are subject to a selection in culture media used for large scale production in order to obtain clones suitable for producing a vaccine intended for human or animal therapy.

4. The method according to any of claims 1 to 3, **characterized in that** the cells originating from the lines obtained in step c) are avian stem cells.

5. The method according to any of claims 1 to 3, **characterized in that** the cells originating from the lines obtained in step c) are avian embryonic stem cells.

6. The method according to any of claims 1 to 5, **characterized in that** it comprises at the end of step c) a step for proliferation of the adherent stem cells in the absence of the inactivated feeder layer.

7. The method according to any of claims 1 to 5, **characterized in that** it comprises at the end of step c) a step for proliferation of the non-adherent stem cells originating from the lines obtained in step c) in suspension.

8. The method according to any of claims 1 to 5, **characterized in that** it comprises at the end of step c) a step for proliferation of the non-adherent stem cells originating from the lines obtained in step c) in suspension in a medium without any exogenous growth factors and cytokine.

9. The method according to any of the preceding claims 1 to 8, **characterized in that** the cells originating from the lines obtained in step c) proliferate in a fetal serum medium in proportions of 2%.

10. The method according to any of claims 1 to 9, **characterized in that** the cells originating from the lines obtained in step c) have at least one of the following features:
- a very visible nucleola and very small nucleoplasm,
- an endogenous alkaline phosphatase activity,
- an endogenous telomerase activity,
- a reactivity with specific antibodies selected from the group of the antibodies SSEA-1 (TEC01), SSEA-3 and EMA-1.

11. The method according to any of claims 1 to 10, **characterized in that** the cells used in step a) are cells obtained by suspending cells originating from blastodermal disks of fertilized eggs in a culture medium comprising at least one cytokine, b-FGF and SCF, said cells being sown on a feeder cells layer, incubated and then sampled.

12. The method according to any of claims 1 to 11, **characterized in that** step b) consists in gradual withdrawal of each growth factor added into the medium of step a), comprising a passage in a new medium lacking at least one of said factors and of repeating different successive passages until the medium is lacking the whole of said factors.

13. The method according to any of claims 1 to 12, **characterized in that** it comprises at the end of step c) a step for proliferation of the cells originating from the lines obtained in step c) in a basis medium selected from DMEM, GMEM, HamF12 or McCoy supplemented with different additives such as non-essential amino acids, vitamins and sodium pyruvate.

14. The method according to any of claims 1 to 13, **characterized in that** the cells originating from the lines obtained in step c) are genetically modified so as to produce a substance of interest, notably a polypeptide of interest, an antibody or an attenuated virus.

15. The method according to any of claims 1 to 13, **characterized in that** the cells originating from the lines obtained in step c) are genetically modified so that they support replication of living or attenuated viruses, in particular viruses selected from the group of adenoviruses, hepadnaviruses, herpes viruses, orthomyxoviruses, papovaviruses, paramyxoviruses, picornaviruses, pox viruses, reoviruses and retroviruses.

16. The method according to claim 15, **characterized in that** the replicated viruses on these cells belong to the family of orthomyxoviruses, in particular the influenza virus.

17. The method according to claim 15, **characterized in that** the replicated viruses belong to the family of paramyxoviruses, in particular the viruses of measles, mumps, and German measles.

18. The use of cell lines or cells originating from these cell lines obtained by a method according to any of claims 1 to 17, for producing substances of interest, in particular proteins of therapeutic interest, antibodies.

19. The use of the cell lines or cells originating from these cell lines obtained by a method according to any of claims 1 to 17, for replication of living or attenuated viruses, in particular the viruses taken from the group adenoviruses, circoviridae, coronaviruses, flaviviruses, hepadnaviruses, herpes viruses, orthomyxoviruses, papovaviruses, paramyxoviruses, picornaviruses, pox viruses in particular the Avipox viruses, notably the Fowlpox virus, the Juncopox virus, the Mynahpox virus, the Pigeonpox virus, the Canarypox virus, the Psittacinepox virus, the Quailpox virus, the Sparrowpox virus, the Starlingpox virus and the Turkeypox virus, reoviruses, retroviruses and togaviridae.

20. The use according to claim 19, for producing viruses belonging to the family of orthomyxoviruses, in particular the influenza virus, notably Influenza A, Influenza B and Influenza C viruses.

21. The use according to claim 19, for producing viruses belonging to the family of paramyxoviruses, in particular the viruses of measles, mumps, German measles, respiroviruses and pneumoviruses.

22. The use according to any of claims 19 to 21, for producing living, attenuated or inactivated human or veterinary vaccines.

## Patentansprüche

1. Verfahren zur Herstellung von Vogelzell-Linien, **dadurch gekennzeichnet, dass** es die folgenden Schritte umfasst:
a) Kultivierung von embryonalen Vogelstammzellen in einem Milieu, das umfasst:
- mindestens die Wachstumsfaktoren SCF, IGF-1 und bFGF und mindestens ein Zytokin, das aus dem LIF, dem IL-LI, dem IL-6, dem IL-6R, dem CNTF, dem Onkostatin und dem Cardiotrophin ausgewählt ist,
- einen von STO-Zellen inaktivierten Feeder Layer und
- fötales Kälberserum in einer Konzentration von 12 bis 8 %,
b) Modifizierung des Kulturmilieus nach zirka zwanzig Durchgängen:
- durch schrittweisen Entzug der Wachstumsfaktoren und des Zytokins, oder
- durch schrittweisen Entzug des Feeder Layers, oder
- durch Absenken der Konzentration des fötalen Kälberserums bis zu geringen Relationen von 2 %,
c) Errichtung von adhärierenden oder nicht adhärierenden Zell-Linien, die in der Lage sind, in einem Basismilieu in Abwesenheit von exogenen Wachstumsfaktoren und von Zytokin, von Serum oder des inaktivierten Feeder Layers zu proliferieren, wobei die Errichtung nicht adhärierender Zell-Linien durch Beimpfen durch mindestens 1 x 10⁶ Zellen/ml im bakteriologischen Behälter erzielt wird, gefolgt von mehreren einfachen Verdünnungsdurchgängen.

2. Verfahren nach Anspruch 1, **dadurch gekennzeichnet, dass** die Zellen, die aus den Linien hervorgegangen sind, die in Schritt c) hergestellt wurden, in der Lage sind, mindestens 50 Tage lang zu proliferieren, vorzugsweise mindestens 600 Tage.

3. Verfahren nach Anspruch 1 oder 2, **dadurch gekennzeichnet, dass** die Zellen, die in Schritt c) hergestellt wurden, einer Selektion in Kulturmilieus unterworfen werden, die für die Produktion in großem Maßstab verwendet werden, um Klone zu erhalten, die für die Produktion von Impfstoff für die Behandlung von Mensch oder Tier geeignet sind.

4. Verfahren nach einem der Ansprüche 1 bis 3, **dadurch gekennzeichnet, dass** die Zellen, die aus den Linien hervorgegangen sind, die in Schritt c) hergestellt wurden, Vogelstammzellen sind.

5. Verfahren nach einem der Ansprüche 1 bis 3, **dadurch gekennzeichnet, dass** die Zellen, die aus den Linien hervorgegangen sind, die in Schritt c) hergestellt wurden, embryonale Vogelstammzellen sind.

6. Verfahren nach einem der Ansprüche 1 bis 5, **dadurch gekennzeichnet, dass** es am Ende von Schritt c) einen Schritt der Proliferierung der adhärierenden Stammzellen in Abwesenheit des inaktivierten Feeder Layers umfasst.

7. Verfahren nach einem der Ansprüche 1 bis 5, **dadurch gekennzeichnet, dass** es am Ende von Schritt c) einen Schritt der Proliferierung der nicht adhärierenden Stammzellen umfasst, die aus den Linien hervorgegangen sind, die in Schritt c) in Suspension hergestellt wurden.

8. Verfahren nach einem der Ansprüche 1 bis 5, **dadurch gekennzeichnet, dass** es am Ende von Schritt c) einen Schritt der Proliferierung der nicht adhärierenden Stammzellen umfasst, die aus den Linien hervorgegangen sind, die in Schritt c) in Suspension in einem Milieu ohne exogene Wachstumsfaktoren und Zytokin hergestellt wurden.

9. Verfahren nach einem der vorangehenden Ansprüche 1 bis 8, **dadurch gekennzeichnet, dass** die Zellen, die aus den Linien hervorgegangen sind, die in Schritt c) hergestellt wurden, in einem Milieu aus fötalem Serum in Relationen von 2 % proliferieren.

10. Verfahren nach einem der Ansprüche 1 bis 9, **dadurch gekennzeichnet, dass** die Zellen, die aus den Linien hervorgegangen sind, die in Schritt c) hergestellt wurden, mindestens eines der folgenden Merkmale aufweisen:
- einen gut sichtbaren Nucleolus und ein sehr schwaches Nucleoplasma,
- eine endogene Alkalin-Phosphatase-Aktivität,
- eine endogene Telomerase-Aktivität,
- eine Reaktivität mit spezifischen Antikörpern, die aus der Gruppe der Antikörper SSEA-1 (TEC01), SSEA-3 und EMA-1 ausgewählt sind.

11. Verfahren nach einem der Ansprüche 1 bis 10, **dadurch gekennzeichnet, dass** die Zellen, die in Schritt a) verwendet wurden, Zellen sind, die durch Suspension von Zellen hergestellt wurden, die von den Blastodermscheiben befruchteter Eier in einem Kulturmilieu herrühren, das mindestens ein Zytokin, b-FGF und SCF umfasst, wobei die Zellen auf einem Layer von Nährzellen besamt und inkubiert und danach entnommen wurden.

12. Verfahren nach einem der Ansprüche 1 bis 11, **dadurch gekennzeichnet, dass** Schritt b) in einem schrittweisen Entzug jedes Wachstumsfaktors besteht, der dem Milieu von Schritt a) hinzugefügt wurde, einen Durchgang in einem neuen Milieu ohne mindestens einen der Faktoren umfassend und in verschiedenen aufeinanderfolgenden Durchgängen zu wiederholen, bis das Milieu ohne einen dieser Faktoren ist.

13. Verfahren nach einem der Ansprüche 1 bis 12, **dadurch gekennzeichnet, dass** es am Ende von Schritt c) einen Schritt der Proliferierung der Zellen umfasst, die aus den Linien hervorgegangen sind, die in Schritt c) in einem Basismilieu hergestellt wurden, das aus DMEM, GMEM, HamF12 oder McCoy ausgewählt und mit verschiedenen Zusatzstoffen wie nicht essentiellen Aminosäuren, Vitaminen und Natriumpyruvat supplementiert ist.

14. Verfahren nach einem der Ansprüche 1 bis 13, **dadurch gekennzeichnet, dass** die Zellen, die aus den Linien hervorgegangen sind, die in Schritt c) hergestellt wurden, genetisch derart modifiziert sind, dass eine interessante Substanz produziert wird, vor allem ein interessantes Polypeptid, ein Antikörper oder ein abgeschwächtes Virus.

15. Verfahren nach einem der Ansprüche 1 bis 13, **dadurch gekennzeichnet, dass** die Zellen, die aus den Linien hervorgegangen sind, die in Schritt c) hergestellt wurden, genetisch derart modifiziert sind, dass sie die Replikation lebender oder abgeschwächter Viren unterstützen, insbesondere der Viren, die aus der Gruppe der Adenoviren, der Hepadnaviren, der Herpesviren, der Orthomyxoviren, der Papovaviren, der Paramyxoviren, der Picornaviren, der Poxviren, der Reoviren und der Retroviren ausgewählt sind,

16. Verfahren nach Anspruch 15, **dadurch gekennzeichnet, dass** die auf diesen Zellen replizierten Viren zur Familie der Orthomyxoviren gehören, insbesondere das Grippevirus.

17. Verfahren nach Anspruch 15, **dadurch gekennzeichnet, dass** die replizierten Viren zur Familie der Paramyxoviren gehören, insbesondere das Masern-, Mumps- und Rötelvirus.

18. Verwendung von Zell-Linien oder Zellen aus diesen Zell-Linien, die durch ein Verfahren nach einem der Ansprüche 1 bis 17 hergestellt sind, zur Herstellung von interessanten Substanzen, insbesondere von therapeutisch interessanten Proteinen, von Antikörpern.

19. Verwendung von Zell-Linien oder Zellen aus diesen Zell-Linien, die durch ein Verfahren nach einem der Ansprüche 1 bis 17 hergestellt sind, zur Replikation von lebenden oder abgeschwächten Viren, insbesondere der Viren, die aus der Gruppe der Adenoviren, der Circoviriden, der Coronaviren, der Flaviviren, der Hepadnaviren, der Herpesviren, der Orthomyxoviren, der Papovaviren, der Paramyxoviren, der Picornaviren, der Poxviren, insbesondere der Avipoxviren, vor allem Fowlpox-Virus, Juncopox-Virus, Mynahpox-Virus, Pigeonpox Virus, Canarypox Virus, Psittacinepox Virus, Quailpox Virus, Sparrowpox Virus, Starlingpox Virus und Turkeypox Virs, der Reoviren, der Retroviren und der Togaviridae entnommen wurden.

20. Verwendung nach Anspruch 19 zur Herstellung von Viren, die zur Familie der Orthomyxoviren gehören, insbesondere das Grippevirus, vor allem Influenza A, Influenza B und Influenza C.

21. Verwendung nach Anspruch 19 zur Herstellung von Viren, die zur Familie der Paramyxoviren gehören, insbesondere das Masern-, Mumps- und Rötelvirus, die Respiroviren und Pneumoviren.

22. Verwendung nach einem der Ansprüche 19 bis 21 zur Herstellung von lebenden, abgeschwächten oder inaktivierten Impfstoffen für Mensch oder Tier.
